(19) **Europäisches Patentamt / European Patent Office / Office européen des brevets**

(11) **EP 4 378 888 A1**

(12) ## EUROPEAN PATENT APPLICATION
published in accordance with Art. 153(4) EPC

(43) Date of publication:
**05.06.2024 Bulletin 2024/23**

(21) Application number: **22849482.9**

(22) Date of filing: **26.07.2022**

(51) International Patent Classification (IPC):
*C01B 13/02* (2006.01)     *A61L 2/10* (2006.01)
*A61L 2/20* (2006.01)     *A61L 9/015* (2006.01)
*C01B 13/10* (2006.01)     *H05H 1/24* (2006.01)

(52) Cooperative Patent Classification (CPC):
**A61L 2/10; A61L 2/20; A61L 9/015; C01B 13/02;
C01B 13/10; H05H 1/24**

(86) International application number:
**PCT/JP2022/028763**

(87) International publication number:
**WO 2023/008421 (02.02.2023 Gazette 2023/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.07.2021   JP 2021126238
13.07.2022   JP 2022112515**

(71) Applicant: **CANON KABUSHIKI KAISHA**
**Tokyo 146-8501 (JP)**

(72) Inventors:
• **FURUKAWA, Takumi**
**Tokyo 146-8501 (JP)**

• **YAMAUCHI, Kazuhiro**
**Tokyo 146-8501 (JP)**
• **OZAWA, Masaki**
**Tokyo 146-8501 (JP)**
• **TAKASHIMA, Kenji**
**Tokyo 146-8501 (JP)**
• **GOTO, Mototeru**
**Tokyo 146-8501 (JP)**
• **KANEKO, Shota**
**Tokyo 146-8501 (JP)**

(74) Representative: **TBK**
**Bavariaring 4-6
80336 München (DE)**

(54) **ACTIVE OXYGEN SUPPLY DEVICE AND METHOD FOR TREATMENT WITH ACTIVE OXYGEN**

(57)     Provided is an active oxygen supply device capable of more efficiently supplying active oxygen to the surface of an object to be treated. The active oxygen supply device comprises: a housing having at least one opening; an ozone generator placed within the housing; a means for generating an air flow that contains ozone generated inside the housing by the ozone generator, moves toward the opening and flows out of the housing through the opening; and an ultraviolet ray light source that is arranged so that the ozone-containing air flow flowing out of the housing through the opening can be irradiated with ultraviolet rays, wherein the ultraviolet ray light source decomposes the ozone in the air flow to generate active oxygen, and the air flow containing the active oxygen, that is generated by irradiating the ozone-containing airflow with the ultraviolet rays, is supplied to an object to be treated outside the housing.

Fig.1A     Fig.1B

EP 4 378 888 A1

**Description**

[Technical field]

The present disclosure is directed to an active oxygen supply device and a method for treatment with active oxygen.

[Background Art]

Ultraviolet light and ozone are known as means for sterilizing items. Patent Document 1 discloses a method by which a problem that sterilization using ultraviolet rays is limited to the part of the object to be sterilized that is irradiated with ultraviolet rays is solved by using a sterilization device that includes an ozone supply device, an ultraviolet generating lamp, and a stirring device and stirring active oxygen generated by irradiating ozone with ultraviolet rays generated from the ultraviolet generating lamp to sterilize even the shaded areas of a sample.

[Citation List]

[Patent Document]

[Patent Document 1] Japanese Patent Application Publication No. H01-25865

[Summary of Invention]

[Technical Problem]

When the present inventors investigated the sterilization performance of the sterilization method according to Patent Document 1, there were cases where the sterilization performance was comparable to that of the conventional sterilization method using only ozone. Since it is commonly believed that the sterilization performance of active oxygen far exceeds that of ozone, this study result was unexpected.

One aspect of the present disclosure is aimed at providing an active oxygen supply device capable of more efficiently supplying active oxygen to the surface of an object to be treated, and a method for treatment with active oxygen capable of more efficiently treating the surface of an object to be treated with active oxygen.

[Solution to Problem]

At least one embodiment of the present disclosure provides an active oxygen supply device comprising:

a housing having at least one opening;
an ozone generator located inside the housing;
a means for generating an airflow comprising ozone generated inside the housing by the ozone generator, flowing toward the opening and flowing out of the housing through the opening; and
an ultraviolet light source arranged so that the airflow comprising the ozone and flowing out of the housing through the opening can be irradiated with ultraviolet rays, wherein
the ultraviolet light source decomposes the ozone in the airflow to generate active oxygen, and
the airflow comprising the active oxygen generated by irradiating the airflow comprising the ozone with the ultraviolet rays is supplied to an object to be treated outside the housing.

Further, at least one embodiment of the present disclosure provides an active oxygen supply device comprising:

a housing having at least one opening;
an ozone generator located inside the housing;
a means for generating an airflow comprising ozone generated inside the housing by the ozone generator, flowing toward the opening and flowing out of the housing through the opening; and
an ultraviolet light source arranged so that the airflow comprising the ozone and having flowed out of the housing through the opening can be irradiated with ultraviolet rays, wherein
the ultraviolet light source decomposes the ozone in the airflow to generate active oxygen, and
the airflow comprising the active oxygen generated by irradiating the airflow comprising the ozone with the ultraviolet rays is supplied to an object to be treated outside the housing.

Furthermore, at least one embodiment of the present disclosure provides a method for treatment by which a surface of the object to be treated is treated with active oxygen, the method comprising:

a step of preparing the above active oxygen supply device;
a step of placing the active oxygen supply device and the object to be treated at relative positions such that the surface of the object to be treated is exposed when the airflow is caused to flow out from the opening; and
a step of causing the airflow to flow out from the opening to supply active oxygen to the surface of the object to be treated, and treating the surface of the object to be treated with active oxygen.

[Advantageous Effects of Invention]

According to one aspect of the present disclosure, it is possible to obtain an active oxygen supply device capable of more efficiently supplying active oxygen to the surface of an object to be treated, and a method for treatment with active oxygen capable of more efficiently treating the surface of an object to be treated with active oxygen.

[Brief Description of the Drawings]

[Fig. 1] Fig. 1 is a schematic cross-sectional view of an active oxygen supply device according to one embodiment of the present disclosure.
[Fig. 2] Fig. 2 is a schematic cross-sectional view of an active oxygen supply device according to another embodiment of the present disclosure.
[Fig. 3] Fig. 3 is a schematic cross-sectional view of an active oxygen supply device according to another embodiment of the present disclosure.
[Fig. 4] Fig. 4 is a schematic cross-sectional view of an active oxygen supply device according to another embodiment of the present disclosure.
[Fig. 5] Fig. 5 is a schematic cross-sectional view of an active oxygen supply device according to another embodiment of the present disclosure.
[Fig. 6] Fig. 6 is a schematic cross-sectional view of an active oxygen supply device according to another embodiment of the present disclosure.
[Fig. 7] Fig. 7 is a schematic cross-sectional view of an active oxygen supply device according to another embodiment of the present disclosure.
[Fig. 8] Fig. 8 is a schematic cross-sectional view of an active oxygen supply device used in Example 13.

[Description of Embodiments]

Hereinafter, specific examples of embodiments for carrying out the present disclosure will be described with reference to the drawings. However, the dimensions, materials, shapes, and relative arrangement of the components described in the embodiments should be changed, as appropriate, according to the configuration of the members to which the disclosure is applied and various conditions. That is, the scope of the present disclosure is not intended to be limited to the following embodiments.

In addition, in the present disclosure, the descriptions of "XX or more and YY or less" or "XX to YY" representing numerical ranges mean numerical ranges including the lower and upper limits, which are endpoints, unless otherwise specified. When numerical ranges are stated stepwise, the upper and lower limits of each numerical range can be combined arbitrarily.

Further, "bacteria" as the object of "sterilization" according to the present disclosure refers to microorganisms, and the microorganisms include fungi, bacteria, unicellular algae, viruses, protozoa, and the like, as well as animal or plant cells (stem cells, dedifferentiated cells and differentiated cells), tissue cultures, fused cells obtained by genetic engineering (including hybridomas), dedifferentiated cells, and transformants (microorganisms). Examples of viruses include, for example, norovirus, rotavirus, influenza virus, adenovirus, coronavirus, measles virus, rubella virus, hepatitis virus, herpes virus, HIV virus, and the like. Examples of bacteria include Staphylococcus, Escherichia coli, Salmonella, Pseudomonas aeruginosa, Vibrio cholerae, Shigella, Anthrax, Mycobacterium tuberculosis, Clostridium botulinum, Tetanus, Streptococcus, and the like. Furthermore, examples of fungi include Trichophyton, Aspergillus, Candida, and the like.

Furthermore, active oxygen in the present disclosure includes, for example, free radicals such as superoxide ($\cdot O_2^-$) and hydroxyl radical ($\cdot OH$) generated by decomposition of ozone ($O_3$).

In the following description, components having the same functions are designated by the same numbers in the drawings, and the description thereof may be omitted.

According to the study by the present inventors, the reason why the sterilization performance of the sterilization device according to Patent Document 1 is limited is assumed to be as follows.

In Patent Document 1, ozone is excited and active oxygen with extremely high sterilizing power is generated by irradiating ozone with ultraviolet rays. Here, active oxygen is a general term for highly reactive oxygen species such as superoxide anion radicals ($\cdot O_2^-$) and hydroxyl radical ($\cdot OH$), and due to own high reactivity thereof, it can instantly oxidize and decompose bacteria and viruses.

However, since ozone absorbs ultraviolet rays extremely well, in the sterilization device according to Patent Document 1, the generation of active oxygen is thought to be limited to the vicinity of the ultraviolet ray generating lamp. That is, it is considered that the ultraviolet rays do not sufficiently reach ozone located at positions away from the ultraviolet ray generating lamp, and active oxygen is unlikely to be generated at positions far from the ultraviolet ray generating lamp.

In addition, active oxygen is extremely unstable (the half-lifespan of $\cdot O_2^-$ is $10^{-6}$ sec, and the half-lifespan of $\cdot OH$ is $10^{-9}$ sec which is extremely short) and is quickly converted into stable oxygen and water. Therefore, it is considered difficult to passively fill the inside of the main body of the sterilization device with active oxygen generated near the ultraviolet generating lamp. In other words, it is considered that sterilization by the sterilization method according to Patent Document 1 is substantially performed by ozone. Therefore, it is considered that the sterilization performance by the sterilization method according to Patent Document 1 is comparable to the sterilization performance by the conventional sterilization method using only ozone.

Based on these considerations, the present inventors recognized that when treating objects using active oxygen, which has a short life, it is necessary to actively place the object and surface to be treated in an active oxygen atmosphere. The present inventors conducted studies based on this recognition and have found that the active oxygen supply device described hereinbelow allows the object to be treated to be more actively arranged in an active oxygen atmosphere.

That is, an active oxygen supply device according to one aspect of the present disclosure comprises: a housing having at least one opening; an ozone generator located inside the housing; a means for generating an airflow comprising ozone generated inside the housing by the ozone generator, flowing toward the opening and flowing out of the housing through the opening; and an ultraviolet light source arranged so that the airflow comprising the ozone and flowing out of the housing through the opening can be irradiated with ultraviolet rays, wherein the ultraviolet light source decomposes the ozone in the airflow to generate active oxygen, and the airflow comprising the active oxygen generated by irradiating the airflow comprising the ozone with the ultraviolet rays is supplied to an object to be treated outside the housing.

Further, an active oxygen supply device according to another aspect of the present disclosure comprises: a housing having at least one opening; an ozone generator located inside the housing; a means for generating an airflow comprising ozone generated inside the housing by the ozone generator, flowing toward the opening and flowing out of the housing through the opening; and an ultraviolet light source arranged so that the airflow comprising the ozone and having flowed out of the housing through the opening can be irradiated with ultraviolet rays, wherein the ultraviolet light source decomposes the ozone in the airflow to generate active oxygen, and the airflow comprising the active oxygen generated by irradiating the airflow comprising the ozone with the ultraviolet rays is supplied to an object to be treated outside the housing.

In the present disclosure, the means for generating an airflow is defined as a means capable of actively causing air to flow out of the housing through the opening even when the active oxygen supply device according to the present disclosure is arranged so that the opening faces vertically upward.

In addition, in the present disclosure, "treatment" of the object to be treated with active oxygen is inclusive of any treatment that can be accomplished with active oxygen, such as surface modification (hydrophilization treatment) sterilization, deodorization, bleaching, and the like of the surface of the object to be treated with active oxygen.

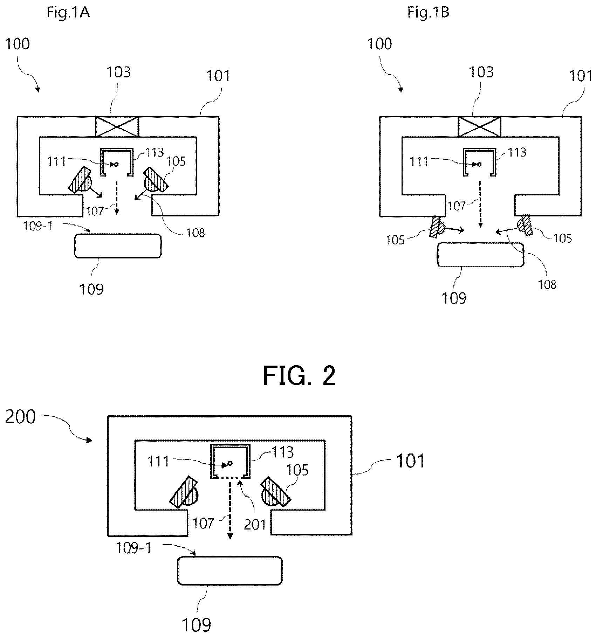

Hereinafter, an active oxygen supply device 100 according to one embodiment of the present disclosure will be described using Fig. 1A.

An active oxygen supply device 100 according to an embodiment of the present disclosure includes a light-emitting diode (LED) 105 as an ultraviolet light source, and a corona discharger as an ozone generator composed of a discharge wire 111, and a shield 113 in a housing 101 having at least one opening. Further, the housing 101 is provided with a fan 103 as a means for drawing air into the housing from outside the housing and generating an airflow flowing out from an opening inside the housing.

An airflow 107 can be generated by generating ozone inside the housing by operating the corona discharger and by introducing air from outside the housing into the housing by using the fan 103. The airflow 107 flows inside the housing to the opening while including ozone generated inside the housing and flows out of the housing from the opening. By irradiating the ozone in the airflow 107 flowing out of the housing with ultraviolet rays from the LED 105, active oxygen can be comprised in the airflow 107 flowing out from the opening. The airflow comprising active oxygen can be supplied to the object to be treated outside the housing. Therefore, active oxygen can be efficiently and actively supplied to the object to be treated that is arranged close to the opening. As a result, a treatment surface 109-1 of an object 109 to be treated is treated with active oxygen. Moreover, since the airflow comprising active oxygen can be supplied to the object to be treated outside the housing, the degree of freedom in selecting the target to which active oxygen is supplied is increased. It becomes possible to move the housing to the desired position for treatment using active oxygen and perform the treatment at this position.

In the active oxygen supply device shown in Fig. 1A, the ultraviolet light sources 105 arranged inside the housing radiate ultraviolet rays onto an airflow flowing from inside the housing toward the opening and flowing out of the housing from the opening. However, the position of the ultraviolet light sources 105 is not particularly limited. That is, the ultraviolet light sources 105 may be arranged inside the housing (hereinafter also referred to as in the interior of the housing), outside the housing, or in the interior of the housing and outside the housing.

When the ultraviolet light source 105 is arranged in the interior of the housing, the ultraviolet light source 105 may be arranged at a position where the airflow flowing out from inside the housing to outside the housing through the opening can be irradiated with ultraviolet rays. Further, when the ultraviolet light source 105 is arranged outside the housing, the ultraviolet light source 105 may be arranged at a position where the airflow that has flowed out from inside the housing to outside the housing through the opening can be irradiated with ultraviolet rays. Furthermore, in the case where the ultraviolet light sources 105 are arranged at least one of in the interior of the housing and on the outside of the housing, the ultraviolet light sources 105 may be arranged so that both the airflow flowing out from the opening to the outside of the housing and the airflow that has flowed out from the opening to the outside of the housing can be irradiated with ultraviolet rays.

More specifically, for example, as shown in Fig. 1B, ultraviolet light sources 105 can be provided outside the housing 101. In such an embodiment, the airflow flowing out from inside the housing to outside the housing through the opening can be more reliably irradiated with ultraviolet rays. Therefore, active oxygen can be generated at a position closer to the object 109 to be treated.

In the embodiment shown in Fig. 1B, the ultraviolet light sources 105 are preferably arranged so that the interior of the housing 101 is not irradiated. Specifically, it is preferable to arrange the ultraviolet light sources 105 so that optical axes 108 thereof do not pass through the interior of the housing 101. This is because active oxygen of higher concentration can be generated at a position closer to the object to be treated 109. In the figure, an arrow 108 from the ultraviolet light source 105 indicates the direction of the optical axis of the ultraviolet light source.

In the active oxygen supply devices according to Figs. 1A and 1B, for example, even when the opening of the active oxygen supply device is disposed facing vertically upward, the fan 103 can introduce the air from outside the housing into the housing so that the airflow containing ozone inside the housing could be actively caused to flow out from the opening. It is more preferable that the fan 103 could produce an airflow such that when the opening of the active oxygen supply device is disposed facing vertically upward, the airflow containing ozone inside the housing is caused to flow out of the housing from the opening at a flow velocity of at least 0.1 m/sec or more, in particular, 2.0 m/sec or more.

<Ultraviolet Light Source and Ultraviolet Rays>

Since the lifespan of active oxygen is extremely short, for example, about one millionth of a second, it is preferable to generate active oxygen very close to the object to be treated from the viewpoint of improving treatment efficiency. Therefore, as the ultraviolet light source, it is preferable to use an LED or a semiconductor laser that can emit strongly directional ultraviolet light. By using a light source capable of emitting highly directional ultraviolet light, it is possible to selectively decompose ozone in the airflow immediately before the airflow flows out of the housing from the opening or immediately after the airflow flows out of the housing from the opening. Therefore, active oxygen can be contained in a higher concentration in the airflow flowing out from the opening. As a result, by arranging the surface 109-1 to be treated near the opening, the surface to be treated can be treated with active oxygen at a high concentration.

In addition, LEDs and semiconductor lasers can selectively radiate ultraviolet rays to the airflow immediately before it flows out of the housing or immediately after it has flowed out, so even if the irradiation output is low, effective supply of active oxygen is likely to be achieved. Furthermore, by using an LED or a semiconductor laser, the device can be made compact and easily configured, for example, as a handy active oxygen supply device that can be held in the user's hand. More preferably, the ultraviolet light source is an LED.

The ultraviolet light source is not particularly limited as long as it has the wavelength and illuminance of ultraviolet rays necessary to excite ozone and obtain an effective active oxygen concentration or effective active oxygen amount corresponding to the purpose of the treatment.

For example, since the peak value of the optical absorption spectrum of ozone is 260 nm, the peak wavelength of the ultraviolet rays emitted by the ultraviolet light source is preferably 220 nm to 310 nm, more preferably 253 nm to 285 nm, and even more preferably 253 nm to 266 nm. When using an LED as an ultraviolet light source, the wavelength is preferably selected from 265 nm, 275 nm, or 280 nm from the viewpoint of output performance.

The relative position of the active oxygen supply device and the object to be treated is not particularly limited. It suffices if at least one of them is arranged so that active oxygen is generated in the airflow flowing out of the housing from the opening, and the surface of the object to be treated is exposed to the airflow in which the effective active oxygen concentration or effective active oxygen amount is maintained according to the purpose of the treatment.

Further, the ultraviolet light source may be arranged at a position where the surface of the object to be treated can be irradiated with ultraviolet rays or may be arranged at a position where the surface of the object to be treated cannot be irradiated with ultraviolet rays. Even if the surface of the object to be treated cannot be irradiated with ultraviolet rays from the ultraviolet light source, the active oxygen supply device according to the present embodiment can treat the object by exposing the surface to be treated to active oxygen in the airflow.

Furthermore, it is preferable that the optical axis 108 of the ultraviolet light source 105 be directed toward the opening through which the airflow comprising ozone flows out. Thereby, it is possible to selectively decompose ozone in the airflow immediately before the airflow flows out of the housing, and to more efficiently supply active oxygen to the object to be treated outside the housing. Furthermore, by directing the optical axis toward the opening, airflow flowing from inside the housing to outside the housing through the opening can be also irradiated by ultraviolet rays. Therefore, it is easier to efficiently supply active oxygen to the surface of the object to be treated. That is, as shown in Fig. 1A, it is preferable that the ultraviolet light source 105 arranged inside the housing 101 be arranged so that the optical axis 108 thereof is directed toward the opening of the housing 101. Furthermore, a configuration is preferable such that the ozone generating means is not irradiated with the ultraviolet rays from the ultraviolet light source 105.

The number of ultraviolet light sources 105 in the active oxygen supply device is not particularly limited, and at least one ultraviolet light source 105 can be provided. The number of ultraviolet light sources 105 may be one, two as in Fig. 1A, or three or more. When two or more ultraviolet light sources 105 are provided, the optical axes of the plurality of ultraviolet light sources 105 can be overlapped at desired positions of the airflow flowing out from the opening or the airflow that has flowed out from the opening. This makes it easier to generate active oxygen at a freely determined position near the surface of the object to be treated.

Furthermore, in the sterilization treatment using ultraviolet rays, only the surface that is irradiated with ultraviolet rays is sterilized. However, in the sterilization treatment using the active oxygen supply device according to the present disclosure, it is possible to sterilize bacteria present at a position that can be reached by active oxygen. Therefore, for example, even bacteria present between fibers that are difficult to sterilize by external ultraviolet irradiation can be sterilized.

Meanwhile, it is preferable that the arrangement be such that the surface of the object to be treated outside the housing could be irradiated through the opening by the ultraviolet rays from the ultraviolet light source. In this case, undecomposed ozone present in the airflow that has flowed out of the housing from the opening can be decomposed in situ on the surface to be treated, and active oxygen can be generated on the surface to be treated. As a result, the degree of treatment and treatment efficiency can be further improved.

In this case, the illuminance of the ultraviolet rays on the surface of the object to be treated or the illuminance of the ultraviolet rays at the opening is not particularly limited, but it is preferable to set the illuminance of the ultraviolet rays, for example, such that ozone contained in the airflow can be decomposed, active oxygen can be generated in the airflow, and an effective active oxygen concentration or effective active oxygen amount corresponding to the purpose of the treatment can be generated on the surface of the object to be treated or at the opening as well.

Specifically, for example, the illuminance of ultraviolet rays on the surface of the object to be treated or the illuminance of ultraviolet rays at the opening is preferably 40 $\mu$W/cm$^2$ or more, more preferably 100 $\mu$W/cm$^2$ or more, and even more preferably 400 $\mu$W/cm$^2$ or more. The upper limit of the illuminance is not particularly limited, but may be, for example, 10,000 $\mu$W/cm$^2$ or less. Within the above ranges, a sufficient amount of active oxygen can be generated in the airflow and supplied to the object to be treated outside the housing.

Furthermore, the distance between the ultraviolet light source and the surface of the object to be treated varies depending on the purpose of the treatment, so it cannot be uniquely defined, but it is preferably, for example, 10 mm or less, and more preferably 4 mm or less. However, it is not necessary to place the object to be treated so that the surface to be treated is at a location within about 10 mm from the ultraviolet light source. The distance between the ultraviolet light source and the object to be treated is not particularly limited as long as the active oxygen in the airflow on the surface of the object to be treated can be adjusted to an effective concentration corresponding to the purpose of the treatment in relation to the illuminance of the ultraviolet rays and the like.

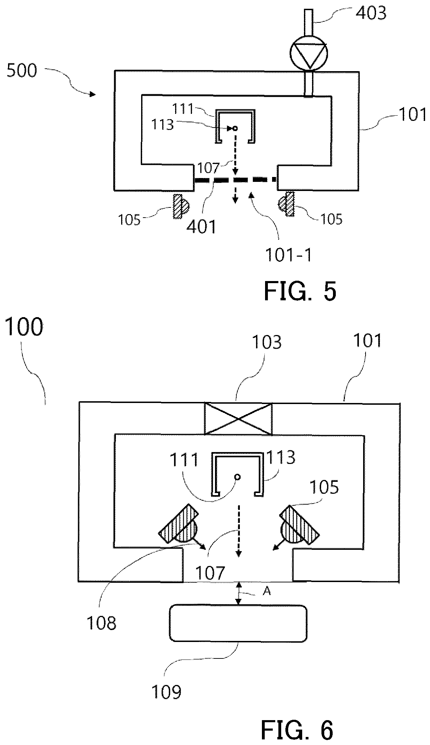

From the same viewpoint, when an ultraviolet light source is provided inside the housing, the distance between the ultraviolet light source and the exit of the opening of the housing is preferably, for example, 10 mm or less, more preferably 4 mm or less. In addition, when treating the object to be treated with the active oxygen supply device, the distance A between the active oxygen supply device and the surface of the object to be treated is preferably, for example, 10 mm or less, more preferably 4 mm or less, and even more preferably 2 mm or less. For example, as shown in Fig. 6, the distance A is the distance between the tip of the opening on the object side and the surface to be treated.

In the active oxygen supply device shown in Fig. 1, a corona discharger was used as the ozone generator, but the ozone generator is not limited to the corona discharger. For example, well-known ozone generators such as other ozone generators (ozonizers) may also be used.

It is preferable that the amount of ozone generated per unit time in the ozone generator without irradiation with ultraviolet rays be, for example, 2 μg/min or more. More preferably, it is 4 μg/min or more, and still more preferably 5 μg/min or more. Within these ranges, a sufficient amount of active oxygen can be generated in the airflow and supplied to the outside of the housing. The upper limit of the amount of ozone generated is not particularly limited, but is, for example, 1000 μg/min or less.

The amount of ozone generated can be controlled by the voltage applied to the ozone generator. The voltage applied to the ozone generator is not particularly limited, but may be, for example, 5 V to 20 V DC.

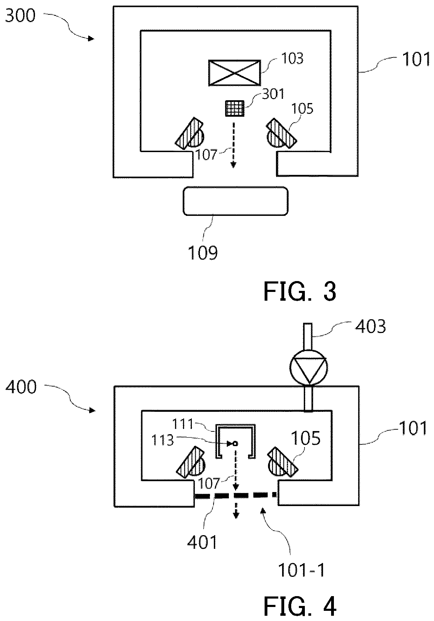

Furthermore, in the configurations of the active oxygen supply devices shown in Figs. 1A and 1B, the fan 103 is built into the housing 101 and the air outside the housing 101 can be taken into the housing 101, but the arrangement of the fan 103 as a means for generating the airflow (airflow generating device) is not limited to this configuration. For example, as shown in Fig. 3, the fan may be arranged on the opposite side of the opening of the ozone generator 301 inside the housing.

The positional relationship between the means for generating airflow, the ozone generator, and the opening is not particularly limited as it depends on the configuration of the means for generating airflow, but it is preferable that the ozone generator be present between the means for generating airflow and the opening. Preferably, the means for generating airflow is oriented such that the airflow could be generated toward the opening. Moreover, it is preferable that the means for generating airflow, the ozone generator, and the opening be arranged in a straight line.

The active oxygen supply device 300 according to another embodiment of the present disclosure shown in Fig. 3 is an example in which an ozonizer is used as the ozone generator 301, but other ozone generators such as a corona discharger can also be used.

Furthermore, the means for generating airflow is not limited to a fan, and a known air blower such as a blower or an air compressor can be used. A plurality of means for generating airflow such as a pump and a grid electrode, which will be described hereinbelow, may be used in combination in addition to the air blower. Preferably, the means for generating the airflow is integrated into or provided inside the housing. Preferably, the air blower includes a means for drawing air into the housing from outside the housing.

An active oxygen supply device 200 according to another embodiment of the present disclosure shown in Fig. 2 does not include the fan 103 as an airflow generating device in the active oxygen supply device 100 shown in Fig. 1. In Fig. 2, the device that generates the airflow flowing out from inside the housing is a grid electrode 201 arranged closer to the opening than the discharge wire 111 of the corona discharger. Further, a power source (not shown) is connected to each of the discharge wire 111 and the grid electrode 201.

By applying a high voltage to the discharge wire 111 and the grid electrode 201 to generate a corona discharge, it is possible to generate an airflow 107 including ozone from the discharge wire 111 toward the grid electrode 201. Moreover, since the grid electrode 201 is arranged on the opening side of the discharge wire 111, the airflow 107 becomes an airflow flowing out of the housing from the opening.

That is, in this configuration, the discharge wire 111 and the grid electrode 201 serve as means for generating an airflow that can actively cause the air inside the housing to flow out from the opening. In this configuration as well, even when the discharge wire opening is arranged vertically upward, it is preferable to produce an airflow such that the airflow containing ozone inside the housing is caused to flow out of the housing from the opening at a flow velocity of at least 0.1 m/sec or more, in particular, 2.0 m/sec or more. In this configuration, it is possible to adjust the flow velocity of the airflow by adjusting the voltage applied between the discharge wire 111 and the grid electrode 201.

By irradiating this airflow 107 with ultraviolet light from the LED 105, active oxygen can be comprised in the airflow 107 flowing out from the opening. Therefore, active oxygen can be supplied to the surface 109-1 to be treated of the object 109 to be treated that is arranged close to the opening. As a result, the treatment surface 109-1 of the object 109 to be treated is treated with active oxygen.

In the configuration of an active oxygen supply device 400 according to another embodiment of the present disclosure shown in Fig. 4, a mesh member 401 is arranged in the opening 101-1 of the housing 101, and when air inside the housing flows out of the housing through the opening, the air encounters flow resistance. In addition, the active oxygen supply device 400 includes an air pump 403 and is configured so that air can be introduced into the housing from outside the housing to create positive pressure inside the housing.

In this active oxygen supply device, even if the air pump 403 is operated to inject air into the housing, since the outflow of air from inside the housing to outside the housing that has passed through the opening encounters flow resistance caused by the mesh member 401 provided in the opening 101-1, positive pressure can be created inside the housing. Therefore, by injecting air into the housing with the air pump 403 and creating a positive pressure inside the housing, an airflow can be generated inside the housing from inside the housing toward the opening. Therefore, when the corona discharger is operated to generate ozone inside the housing, the airflow 107 that includes ozone is generated inside the housing, flows toward the opening, and flows out of the housing from the opening.

Then, by irradiating the ozone in the airflow 107 flowing out of the housing with ultraviolet rays from the LED 105, active oxygen can be comprised in the airflow 107 flowing out from the opening. Therefore, active oxygen can be supplied to the object to be treated that is arranged close to the opening. As a result, similarly to the case of the active oxygen supply device shown in Fig. 1, the treatment surface of the object to be treated is treated with active oxygen.

In Fig. 4, the mesh member 401 is used, but any ozone permeable member that can create positive pressure inside the housing and can allow airflow to flow out from the opening can be provided in the opening 101-1. Examples thereof include, a porous plate such as a punching member, a porous membrane, a nonwoven fabric, a woven fabric, a net, and the like. The material of the ozone permeable member is preferably a material that has low ozone-induced corrosiveness, and examples thereof include metals such as stainless steel, silver, gold, and aluminum, nonmetallic inorganic materials such as glass and ceramics, resins such as fluororesin, and the like.

The shape of the ozone permeable member is not particularly limited as long as it can create a positive pressure inside the housing and generate the airflow 107. For example, the wire diameter of the ozone permeable member 401 is preferably 0.01 mm to 2 mm, more preferably 0.1 mm to 0.5 mm. The aperture ratio of the ozone permeable member 401 is preferably 1% to 50%, more preferably 5% to 40%.

In the case of a perforated plate, the hole diameter is preferably 0.01 mm to 2 mm, and the aperture ratio is preferably 1% to 50%, more preferably 5% to 40%.

The material of the ozone permeable member is not particularly limited, and well-known metal or resin may be used. From the viewpoint of enabling the irradiation of the surface of the object to be treated with ultraviolet rays, it is preferable to use an ultraviolet-transparent resin.

In addition, in the active oxygen supply device shown in Fig. 4, the airflow flowing from inside the housing toward the opening and flowing out of the housing from the opening is irradiated from the ultraviolet light source 105 arranged inside the housing. However, similar to Fig. 1B, the ultraviolet light source 105 may be configured to irradiate the airflow that has flown out from inside the housing through the opening to outside the housing with ultraviolet rays, as shown in Fig. 5. In this case, it is possible to decompose ozone in the airflow 107 and generate active oxygen at a position closer to the object to be treated, so it is thought that the treatment efficiency of the surface to be treated can be further improved. In addition, in this case, the material of the mesh member or the punching member is preferably a material, such as metal, that does not easily transmit ultraviolet rays, so that the ultraviolet rays do not easily reach the ozone inside the housing. As the material, it is more preferable to use a material that has a high reflectance of ultraviolet rays, for example, such as aluminum with a reflectance of 80% or more, since the object to be treated can be irradiated with scattered ultraviolet light.

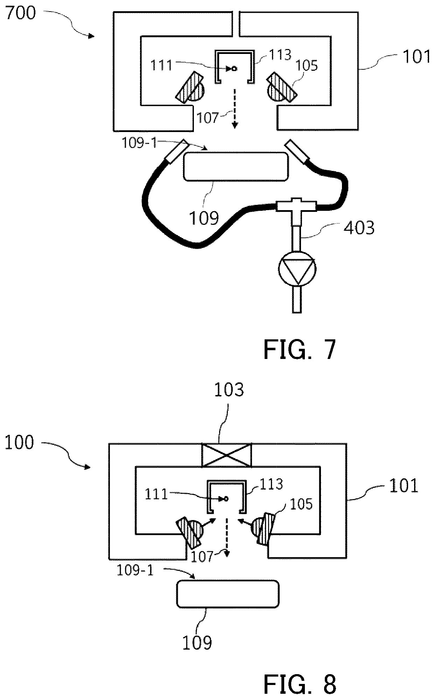

It is also a preferred embodiment that the means for generating the airflow is a suction device for gas inside the housing. An active oxygen supply device 700 according to another embodiment of the present disclosure shown in Fig. 7 includes the air pump 403 outside the housing 101 as a suction device for gas inside the housing. An air pump intake port is provided near the opening to be able to suck the gas inside the housing. Furthermore, a ventilation hole is provided on the back side of the opening in the housing 101, so that the air inside the housing flows out of the housing through the opening.

In this active oxygen supply device, by releasing gas near the opening with the air pump 403 and sucking in atmospheric air through the vent at the top of the housing, it is possible to generate an airflow from inside the housing toward the opening. Therefore, when the corona discharger is operated to generate ozone inside the housing, the airflow 107 comprising ozone inside the housing flows toward the opening and flows out of the housing from the opening.

By irradiating the ozone in the airflow 107 flowing out of the housing with ultraviolet light from the LED 105, active oxygen can be comprised in the airflow 107 flowing out from the opening.

Therefore, active oxygen can be actively supplied to the object to be treated that is arranged close to the opening. As a result, similarly to the case of the active oxygen supply device shown in Fig. 1, the surface of the object to be treated is treated with active oxygen.

In this configuration as well, even when the opening provided with the air pump intake port is arranged vertically upward, it is preferable to create an airflow such that the airflow comprising ozone inside the housing is caused to flow out from the opening to outside the housing at a flow velocity of at least 0.1 m/sec or more, in particular, 2.0 m/sec or more. In this configuration, it is possible to adjust the flow velocity of the airflow by adjusting the suction force of the air pump, the direction of the intake port, the size of the opening, the size of the vent, and the like.

In the active oxygen supply device according to each of the embodiments shown in Figs. 1 to 7, the flow velocity at the outlet of the opening of the airflow flowing out of the housing from the opening or the airflow that has flown out of the housing from the opening is preferably 0.1 m/sec to 100 m/sec, more preferably 1.0 m/sec to 10.0 m/sec, even more preferably 2.0 m/sec to 9.5 m/sec, and still more preferably 3.0 m/sec to 9.0 m/sec regardless of the orientation of the opening. Specifically, for example, the flow velocity of the airflow measured at the outlet of the opening when the active oxygen supply device is arranged so that the opening thereof faces vertically upward is preferably 0.1 m/sec to 100 m/sec, more preferably 1.0 m/sec to 10.0 m/sec, still more preferably 2.0 m/sec to 9.5 m/sec, and even more preferably 3.0 m/sec to 9.0 m/sec.

Where the flow velocity of the airflow is within the above ranges, active oxygen with a high decomposition rate can be more actively supplied to the outside of the housing. Furthermore, since the airflow comprising ozone can be efficiently caused to flow relative to the ultraviolet light source, active oxygen can be more efficiently supplied to the object to be treated.

The flow velocity of the airflow can be controlled, as appropriate, depending on the means for generating the airflow. If a fan, a pump, a blower, or a compressor is used, control may be performed by operation intensity thereof or the like, and if a grid electrode is used, control may be performed by applied voltage or the like. Additionally, the flow velocity of the airflow can also be controlled by the size of the housing and the opening. The flow velocity of the airflow at the outlet of the opening can be measured using a hot wire anemometer, a propeller anemometer, a PIV, or the like.

The shape of the opening is not particularly limited. The shape may be freely selected from a rectangle, a square, a circle, an ellipse, or a partially deformed shape thereof, such as a substantially rectangular shape, a substantially square shape, a substantially circular shape, a substantially elliptical shape, and the like. Where uniformity of treatment is desired, a circular shape is preferred as it provides high uniformity of airflow within the opening.

The size of the opening is not particularly limited and may be changed, as appropriate, depending on the application of the active oxygen supply device, the size of the supply target, and the like. For example, the area of the opening is preferably about 5 cm$^2$ to 2000 cm$^2$, more preferably about 10 cm$^2$ to 100 cm$^2$.

From the viewpoint of increasing the flow velocity of airflow and supplying active oxygen to the outside of the housing more actively, the area of the opening is preferably smaller than the maximum value (area H) of the area of the cross section inside the housing parallel to the plane forming the opening. The ratio of the maximum value (area H) of the area of the cross section inside the housing parallel to the plane forming the opening to the area of the opening (area H/area of the opening) is preferably 1.5 to 20, more preferably 2 to 15, even more preferably 3 to 10, and still more preferably 4 to 7.

The active oxygen supply device of the present disclosure can be used not only for the purpose of sterilizing the objects to be treated, but also for all applications implemented by supplying active oxygen to objects to be treated. For example, the active oxygen supply device of the present disclosure can be used for the purpose of deodorizing the object to be treated, the purpose of bleaching the object to be treated, hydrophilization surface treatment of the object to be treated, and the like.

In addition, the active oxygen supply device of the present disclosure can be used not only for performing the treatment of sterilizing the object to be treated, but also, for example, for performing the treatment of deodorizing the object to be treated, the treatment of bleaching the object to be treated, the surface treatment that hydrophilizes the object to be treated, and the like.

In addition, in the present disclosure, "effective active oxygen concentration or effective active oxygen amount" refers to the active oxygen concentration or active oxygen amount for achieving the purpose of treating the object, for example, such as sterilization, deodorization, bleaching, or hydrophilization and can be adjusted, as appropriate, according to the purpose by using the amount of ozone generated per unit time in the ozone generator, the irradiation position, illuminance and irradiation time of ultraviolet rays, the flow velocity of airflow, and the like.

Additionally, the present disclosure provides a method for treatment by which the surface of an object to be treated is treated with active oxygen.

The treatment method includes: a step of preparing the above-described active oxygen supply device;

a step of placing the active oxygen supply device and the object to be treated at relative positions such that the surface of the object to be treated is exposed when the airflow is caused to flow out from the opening; and

a step of causing the airflow to flow out from the opening to supply active oxygen to the surface of the object to be treated, and treating the surface of the object to be treated with active oxygen.

The treatment time may be set, as appropriate, according to the purpose, such as sterilization, deodorization, bleaching, or hydrophilization. For example, the treatment time is about 1 sec to 1000 min, preferably about 5 sec to 150 min.

EXAMPLES

Hereinafter, the present disclosure will be described in more detail using Examples and Comparative Examples, but the embodiments of the present disclosure are not limited thereto.

<Example 1>

The active oxygen supply device of Example 1 is shown in Fig. 1A. As the housing 101 of the active oxygen supply device 100, an aluminum case of rectangular parallelepiped shape with the inner dimensions of 50 mm in height, 100 mm in width, and 100 mm in depth was prepared. A fan 103 was provided on the top surface, and a circular opening with a diameter of 50 mm was provided on the bottom surface, allowing airflow to flow out from the opening.

Additionally, four ultraviolet light sources 105 (UV-C LED, trade name: ZEUBE265-2CA, manufactured by Stanley Electric Co., Ltd., peak wavelength = 265 nm) were arranged with a 90° interval of the circular shape inside the housing. In addition, a corotron corona discharger (DC-8 kV applied), which is used as a charging member of an electrophotographic apparatus and is composed of a discharge wire 111 and a shield 113, was installed as an ozone generator in the center of the upper surface of the housing. In this way, the active oxygen supply device according to this Example was produced. Further, the arrangement was such that the distance A between the bottom surface of the housing 101 and the surface 109-1 to be treated of the object 109 to be treated in Fig. 6 was 1 mm. The optical axis of the LED passed through the aperture toward the surface 109-1 to be treated so that the surface to be treated 109-1 could be irradiated.

The illuminance of ultraviolet rays was measured by placing the light receiving part of an illuminance meter (product name: Spectral Irradiance Meter USR-45D, manufactured by Ushio Inc.) so that the light receiving surface thereof coincided with the surface to be treated that is the active oxygen generation position in the active oxygen supply device 100. The integral value of the spectrum obtained by applying a voltage of 7 V to the ultraviolet light source 105 was 630 $\mu$W/cm$^2$. At this time, the ozone generator was not powered on so as to avoid the influence of the shielding of ultraviolet rays by ozone generated from the ozone generator. The illuminance of ultraviolet rays measured under these conditions was regarded as the illuminance of ultraviolet rays that contribute to the excitation of ozone on the surface to be treated.

Subsequently, in order to calculate the amount of ozone generated from the ozone generator, the opening of the housing 101 and the fan 103 of the active oxygen supply device 100 were covered with an ozone-impermeable lid to form a closed container. Then, a hole (not shown) that could be sealed with a rubber stopper was provided in a part of the lid which was placed on the opening, so that the gas inside could be sucked through the hole with a syringe. Then, with a DC voltage of -8 kV applied to the discharge wire 111 and the shield 113 grounded, 100 ml of the gas in the closed container was sampled one minute later. The sampled gas was sucked into an ozone detection tube (trade name: 182SB, manufactured by Komyo Rikagaku Kogyo K.K.), and the measured ozone concentration (PPM) generated from the ozone generator was measured. Using the measured ozone concentration value, the amount of ozone generated per unit time was determined by the following formula.

[Math. 1]

$$Amount\ of\ ozone\ generated\ per\ unit\ time\ (\frac{mg}{min}) =$$

$$Measured\ ozone\ concentration(PPM) * \frac{Molecular\ weight\ of\ ozone\ 48}{22.4}$$

$$* \frac{\frac{273}{273 + Room\ temperature\ (°C)}}{10000} * \frac{Gas\ in\ container\ (L)}{Sampled\ gas\ (L)}$$

$$= Measured\ ozone\ concentration\ (PPM) * 48/22.4 * 273/(273 + 25)/10000 * 0.5/0.1$$

As a result, the amount of ozone generated per unit time was 5 $\mu$g/min. At this time, the ultraviolet light source was not powered on so as to avoid the influence of ozone decomposition due to ultraviolet light emitted from the ultraviolet light source.

Finally, the amount of ozone generated when both the ozone generator and the ultraviolet light source 105 were in operation was measured. The operating conditions for the ozone generator were such that 5 $\mu$g/min of ozone was generated when only the ozone generator was in operation. Further, the operating conditions for the ultraviolet light source 105 were such that when only the ultraviolet light source 105 was in operation, the irradiation intensity was 630 $\mu$W/cm$^2$. As a result, the amount of ozone generated when both the ozone generator and the ultraviolet light source 105 were in operation was 2 $\mu$g/min. It is considered that the decrease of 3 $\mu$g/min from 5 $\mu$g/min is the amount of ozone converted to active oxygen.

2-1. Treatment (Hydrophilization) Test

A polypropylene resin plate (manufactured by TP Giken Co., Ltd.) cut into a 20 mm square was used and arranged as the object 109 to be treated so that the distance A in Fig. 6 of the active oxygen supply device 100 was 1 mm. Next, a voltage of -8 kV DC was applied to the discharge wire 111, a voltage of 7 V DC was applied to the ultraviolet light source 105 to radiate ultraviolet rays, and treatment was performed for 2 h. The rotation speed of the fan 103 was adjusted so that when the active oxygen supply device was arranged with the opening facing vertically upward, the flow velocity of the airflow at the opening was 8.2 m/sec.

Thereafter, the contact angle of the surface of the polypropylene resin plate treated with active oxygen with respect to water was measured and compared with the contact angle before the treatment. The contact angle was measured at 23°C and 50% RH using an automatic contact angle meter (product name: DMo-602, manufactured by Kyowa Interface Science Co., Ltd.) as a measuring device, and using 0.5 μL of water as a droplet. The angle after 500 milliseconds of dropping was measured, and the average value of the five points was adopted. The contact angle of the surface of the polypropylene resin plate before the treatment was 102°, and the contact angle after the treatment was 77°, so the contact angle decreased by 25°.

2-2. Treatment (Sterilization) Test

Using the active oxygen supply device 100, an E. coli sterilization test was conducted according to the following procedure. All the instruments used in this sterilization test were sterilized using high-pressure steam using an autoclave. In addition, this sterilization test was conducted in a clean bench.

First, in an Erlenmeyer flask containing an LB medium (2 g of tryptone, 1 g of yeast extract, 1 g of sodium chloride, combined with distilled water to make 200 ml), Escherichia coli (product name: "KWIK-STIK (Escherichia coli ATCC 8739)", manufactured by Microbiologies) was added and cultured with shaking at 80 rpm for 48 h at a temperature of 37°C. The E. coli solution after culturing was $9.2 \times 10^9$ (CFU/ml).

Using a micropipette, 0.010 ml of this cultured bacterial solution was dropped onto a slide glass (Matsunami Glass, model number: S2441) measuring 3 cm long, 1 cm wide, and 1 mm thick, and the bacterial solution was applied to the entire surface of one side of the slide glass with the tip of the micropipette to produce sample No. 1. Sample No. 2 was produced in a similar manner.

Sample No. 1 as the object 109 to be treated was arranged so that the distance A in Fig. 6 between the surface of the slide glass to which the bacterial solution was applied (surface to be treated) and the active oxygen supply device 100 was 1 mm.

Next, the active oxygen supply device 100 was activated, a DC voltage of -8 kV was applied to the discharge wire 111, and a DC voltage of 7 V was applied to the ultraviolet light source 105 to radiate ultraviolet rays, and the surface of the slide glass to which the bacterial solution had been applied was treated with the airflow containing active oxygen. The treatment time was 30 sec.

Next, sample No. 1 was immersed in a test tube containing 10 ml of a buffer solution (trade name "Gibco PBS", Thermo Fisher Scientific Inc.) for 1 h. In addition, in order to prevent the bacterial solution on the slide glass from drying during the treatment process using the active oxygen supply device, the time from dropping the bacterial solution onto the slide glass to immersion in the buffer solution was set to 60 sec.

Next, 1 ml of the buffer solution (hereinafter also referred to as "1/1 solution") after immersing sample No. 1 was placed into a test tube containing 9 ml of buffer solution to prepare a diluted solution (hereinafter "1/10 diluted solution"). A 1/100 diluted solution, a 1/1000 diluted solution, and a 1/10,000 diluted solution were prepared in the same manner except that the dilution ratio with the buffer solution was changed.

Next, 0.050 ml was collected from the 1/1 solution and smeared on a stamp medium (Petan Check 25 PT1025 manufactured by Eiken Chemical Co., Ltd.). This operation was repeated to prepare two stamp media (n1, n2) coated with the 1/1 solution. The two stamp media were placed in a thermostat (trade name: IS600; manufactured by Yamato Scientific Co., Ltd.) and cultured at a temperature of 37°C for 24 h.

Similarly to the 1/1 solution, for the 1/10 diluted solution, 1/100 diluted solution, 1/1000 diluted solution, and 1/10000 diluted solution, two smeared stamp media were prepared and cultured for each diluted solution.

Then, among the number of colonies generated for each stamp medium related to each diluted solution, 25(CFU) which was the average value of the number of colonies of the two stamp media related to the 1/1 solution for which the number of colonies was within the range of 10 or more to 100 or less was defined as the number of viable bacteria in 0.050 ml of the 1/1 solution.

[Table 1]

|  | n1 | n2 |
|---|---|---|
| 1/1 Solution | 23 | 27 |
| 1/10 Diluted solution | 1 | 2 |
| 1/100 Diluted solution | 0 | 0 |
| 1/1000 Diluted solution | 0 | 0 |

(continued)

|  | n1 | n2 |
|---|---|---|
| 1/10000 Diluted solution | 0 | 0 |

Next, 1/1 solution, 1/10 diluted solution, 1/100 diluted solution, 1/1000 diluted solution, and 1/10000 diluted solution were prepared and the culture test was conducted was performed for sample No. 2 in the same manner as for sample No. 1, except that the treatment with the active oxygen supply device was not conducted. Then, based on the number of colonies in the two stamp media that became 10 or more and 100 or less, the number of viable bacteria in the 1/1 solution related to sample No. 2 was calculated. As a result, the number of viable bacteria in 0.0050 ml of the 1/1 solution related to sample No. 2 was 595,000 (CFU).

Therefore, the sterilization rate of E. coli by the active oxygen supply device according to this test was 99.996% (= (595,000 - 25)/595,000 × 100).

2-3. Treatment (Deodorization) Test

(1) Preparation of samples for deodorization test

A paper wiper (Kimwipe S-200, manufactured by Nippon Paper Crecia Co., Ltd.) was immersed in Fabric Mist (trade name: Fabric Mist Linen, manufactured by Sabon Co., Ltd.) for 10 min, then taken out and allowed to dry in air for 6 h. Next, the paper wiper was cut into three pieces measuring 10 mm in length and 10 mm in width to obtain samples for the deodorization test.

(2) Deodorization test

One of the three deodorization test samples was installed so that the distance between the opening of the active oxygen supply device 100 and the surface 109-1 to be treated (distance A in Fig. 6) was 1 mm. Furthermore, the center position of the object 109 in the width direction (horizontal direction in Fig. 6) and the depth direction (depth direction in Fig. 6) was placed at the center of the opening when the active oxygen supply device 100 was viewed from the bottom.

Next, a voltage of -8 kV DC was applied to the discharge wire 111, and a voltage of 7 V DC was applied to the ultraviolet light source 105 to radiate ultraviolet rays, thereby irradiating active oxygen for 30 min on the surface to be treated and treating the surface 109-1 to be treated.

Then, the extent to which the odor of the treated sample remained in comparison with the sample that had not been treated with active oxygen was evaluated using the following intensity criteria. The evaluation was conducted on 5 subjects, and the intensity criteria selected by at least 3 subjects were adopted. This was repeated three times, and the three treated samples were compared with the untreated samples to determine the degree of deodorization, and evaluated using the following criteria.

Rank A: Odorless.
Rank B: Odor that can barely be detected (detection threshold).
Rank C: Weak odor (recognition threshold) that can be recognized as the odor of fabric mist.
Rank D: No difference from untreated sample.

2-4. Treatment (Bleaching) Test

(1) Preparation of samples for bleaching test

Chili pepper sauce (trade name: Tabasco Pepper Sauce, manufactured by McIlhenny Co., Ltd.) was filtered through a long fiber nonwoven fabric (trade name: Bencot M-3II, manufactured by Asahi Kasei Corporation) to remove solid fraction. A paper wiper (trade name: Kimwipe S-200, manufactured by Nippon Paper Crecia Co., Ltd.) was immersed in the obtained liquid for 10 min. Next, the paper wiper was taken out and washed with water. Washing with water was repeated until the washing solution was no longer visually observed to be colored. Then, drying was performed. Next, three samples measuring 15 mm in length and 15 mm in width were cut out from the paper wiper dyed red with the chili sauce to obtain samples for bleaching tests.

(2) Bleaching test

One of the three samples for bleaching test was installed so that the distance between the opening of the active oxygen supply device 100 and the surface 109-1 to be treated (distance A in Fig. 6) was 1 mm. Further, the center position of the object 109 in the width direction (horizontal direction in Fig. 6) and the depth direction (depth direction in Fig. 6) was arranged at the center when the active oxygen supply device was viewed from the bottom. Next, a voltage of -8 kV DC was applied to the discharge wire 111, and a voltage of 7 V DC was applied to the ultraviolet light source 105 to radiate ultraviolet rays, thereby irradiating active oxygen for 150 min on the surface to be treated and treating the surface 109-1 to be treated. This was repeated three times, and the three treated samples were compared with the untreated sample to visually observe how much decolorization had occurred and evaluated using the following criteria.

Rank A: Completely bleached.
Rank B: The red color of the chili pepper sauce remained slightly.
Rank C: Some red color from the chili pepper sauce remained.
Rank D: There was no difference in color from the part where active oxygen was not supplied.

Device conditions of the active oxygen supply device of Example 1, the amount of ozone generated when only the ozone generator was operated, the illuminance of ultraviolet rays when only the ultraviolet light source was operated, the decrease in contact angle, and the evaluation results for each treatment in sterilization, deodorization, and bleaching are shown in Table 3.

<Example 2>

An active oxygen supply device was produced and evaluated in the same manner as in Example 1, except that the voltage of the ultraviolet light source 105 in Example 1 was changed from 7 V to 4 V and the UV illuminance was lowered.

<Example 3>

An active oxygen supply device was produced and evaluated in the same manner as in Example 1, except that the voltage applied to the discharge wire 111 in Example 1 was changed to -5 kV DC and the amount of ozone generated was reduced.

<Example 4>

An active oxygen supply device was produced and evaluated in the same manner as in Example 1, except that the ultraviolet light source in Example 1 was changed to a UV-C LED (model number: LJU1106EAE-275-TR, manufactured by Stanley Electric Co., Ltd., peak wavelength = 275 nm).

<Examples 5 and 6>

The active oxygen supply device was evaluated in the same manner as in Example 1, except that the distance A between the lower surface of the housing 101 and the surface 109-1 to be treated was changed as shown in Table 2.

<Example 7>

An active oxygen supply device was produced and evaluated in the same manner as in Example 1, except that the active oxygen supply device in Fig. 1A of Example 1 was changed to the active oxygen supply device in Fig. 1B. In Example 7, the position of the ultraviolet light source 105 was moved from that shown in Fig. 1A to outside the housing 101 as shown in Fig. 1B. The distance between the surface 109-1 to be treated and the lower end of the ultraviolet light source was defined as the distance A between the active oxygen supply device and the surface to be treated. The optical axis of the LED was set toward the surface 109-1 to be treated so that the surface 109-1 to be treated could be irradiated.

<Example 8>

An active oxygen supply device was produced and evaluated in the same manner as in Example 1, except that the active oxygen supply device 100 in Fig. 1A of Example 1 was changed to the active oxygen supply device 200 in Fig. 2. That is, in the active oxygen supply device 200, instead of using a fan as a means for generating airflow, the ozone generator was changed from a corotron corona discharger to a scorotron corona discharger having a grid electrode 201. A DC voltage of 1 kV was applied to the grid electrode 201.

<Example 9>

An active oxygen supply device was produced and evaluated in the same manner as in Example 1, except that the active oxygen supply device 100 in Fig. 1A of Example 1 was changed to the active oxygen supply device 300 in Fig. 3. That is, in the active oxygen supply device 300, a fan was installed inside the housing, and the ozone generator was changed from a corotron corona discharger to the ozonizer 301 (product number: MHM500-00A, manufactured by Murata Manufacturing Co., Ltd.). A DC voltage of 8 V was applied to the ozonizer.

Regarding the sterilization test in this example, as a result of the culture test of the 1/1 solution, 1/10 diluted solution, 1/100 diluted solution, 1/1000 diluted solution and 1/10000 diluted solution related to sample No. 1, since the number of colonies observed in stamp culture media (n1, n2) related to the 1/1 solution was less than 10 in both cases, the sterilization rate was calculated by assuming that the number of viable bacteria in 0.050 ml of 1/1 solution was 10 (CFU).

<Example 10>

An active oxygen supply device was produced and evaluated in the same manner as in Example 1, except that the active oxygen supply device 100 in Fig. 1A of Example 1 was changed to the active oxygen supply device 400 in Fig. 4. That is, in the active oxygen supply device 200, an air pump 403 (model number "β-60", manufactured by As One Corporation, discharge air rate 1.2 L/min) was installed by connection via a silicone tube, instead of using a fan, as a means to generate airflow, and a mesh member 401 (made of stainless steel, 100 mesh, linear 0.1 mm, aperture ratio 36%) was installed in the opening.

<Example 11>

An active oxygen supply device was produced and evaluated in the same manner as in Example 10, except that the active oxygen supply device 400 in Fig. 4 of Example 10 was changed to the active oxygen supply device 500 in Fig. 5. In the active oxygen supply device 500, the position of the ultraviolet light source 105 was moved from that in Fig. 4 to outside the housing 101 as shown in Fig. 5. The distance between the surface 109-1 to be treated and the lower end of the ultraviolet light source was defined as the distance A between the active oxygen supply device and the surface to be treated. The optical axis of the LED was set toward the surface 109-1 to be treated so that the surface 109-1 to be treated could be irradiated.

<Example 12>

An active oxygen supply device was produced and evaluated in the same manner as in Example 1, except that the active oxygen supply device 100 in Fig. 1A of Example 1 was changed to the active oxygen supply device 700 in Fig. 7. That is, in the active oxygen supply device 700, an air pump 403 (model number "β-60", manufactured by As One Corporation, discharge air rate 1.2 L/min) was installed outside the housing by connection via a silicone tube, instead of using a fan, as a means to generate airflow, and the tip of the silicone tube that served as the intake port was installed near the opening between the opening and the object to be treated. In addition, a vent hole with a diameter of 5 mm was provided on the back side of the opening.

<Example 13>

In the active oxygen supply device used in Example 1, the LED serving as the ultraviolet light source 105 was arranged so that the optical axis thereof faced the ozone generator (discharge wire 111, shield 113) as shown in Fig. 8, and the ozone generator was irradiated by ultraviolet light emitted from the LED. Other than this, the treatment was carried out in the same manner as in Example 1.

<Comparative Examples 1 to 3>

Comparative Examples 1 to 3 were implemented under the same conditions as Example 1, except for the following configurations.

Comparative Example 1: A voltage was applied to the ozone generator, but no voltage was applied to the ultraviolet light source.

Comparative Example 2: No voltage was applied to the ozone generator, but voltage was applied to the ultraviolet light source.

Comparative Example 3: The conditions were the same as in Example 9, except that the fan in Fig. 3 was not driven, so that no airflow was generated.

Table 2 shows the evaluation results of Examples 1 to 13 and Comparative Examples 1 to 3.

[Table 2]

| | | Embodiment of active oxygen supply device | Flow velocity of airflow (m/sec) | Distance A (mm) | UV peak wavelength (nm) | Amount of generated ozone (pg/min) | UV illuminance ($\mu$W/cm$^2$) | Hydrophilization test | Sterilization test | | | Deodorization test | Bleaching test |
|---|---|---|---|---|---|---|---|---|---|---|---|---|---|
| | | | | | | | | Decreased contact angle (°) | Determination of sterilization performance (CFU) | Sterilization rate | Evaluation rank | | |
| Example | 1 | Fig.1A | 8.2 | 1 | 265 | 5 | 630 | 31 | 25 | 99.996% | A | A |
| | 2 | Fig.1A | 8.2 | 1 | 265 | 5 | 250 | 18 | 120 | 99.980% | A | A |
| | 3 | Fig.1A | 8.2 | 1 | 265 | 2.5 | 630 | 23 | 71 | 99.988% | A | A |
| | 4 | Fig.1A | 8.2 | 1 | 275 | 5 | 580 | 29 | 43 | 99.993% | A | A |
| | 5 | Fig.1A | 8.2 | 3 | 265 | 5 | 630 | 23 | 320 | 99.946% | A | A |
| | 6 | Fig.1A | 8.2 | 10 | 265 | 5 | 630 | 11 | 860 | 99.855% | A | A |
| | 7 | Fig.1B | 7.4 | 1 | 265 | 5 | 630 | 30 | 36 | 99.994% | A | A |
| | 8 | Fig.2 | 3.5 | 1 | 265 | 6 | 630 | 28 | 55 | 99.991% | A | A |
| | 9 | Fig.3 | 3.1 | 1 | 265 | 38 | 630 | 31 | 10 | 99.998% | A | A |
| | 10 | Fig.4 | 2.5 | 1 | 265 | 5 | 630 | 17 | 920 | 99.845% | B | B |
| | 11 | Fig.5 | 2.1 | 1 | 265 | 5 | 630 | 25 | 81 | 99.986% | A | A |
| | 12 | Fig.7 | 0.5 | 1 | 265 | 5 | 630 | 22 | 95 | 99.984% | A | A |
| | 13 | Fig.8 | 8.2 | 1 | 265 | 5 | 70 | 12 | 1500 | 99.748% | A | A |
| Comparative Example | 1 | Fig.1A | 8.2 | 1 | 265 | 5 | 0 | 8 | 335000 | 43.697% | C | C |
| | 2 | Fig.1A | 8.2 | 1 | 265 | 0 | 630 | 0 | 8400 | 98.588% | D | D |
| | 3 | Fig.3 | 0.0 | 1 | 265 | 38 | 630 | 31 | 42000 | 92.941% | D | D |

EP 4 378 888 A1

In Table 2, UV represents ultraviolent light. Further, the amount of generated ozone indicates the amount of ozone generated per uunit time when the ultraviolent light source is not powered on. Further, the UV illuminance indicates the UV illuminance when the ozone generator is not powered on. Furthermore, the flow velocity of airflow in the table is a value measured at the opening when the active oxygen supply device according to each embodiment is arranged so that the opening faces vertically upward.

To measure the flow velocity, a multi-environment measuring device (product name: Testo 435; manufactured by Testo SE & Co.) was used, the center of the anemometer probe was aligned with the center of the opening, and the flow velocity of the airflow was measured immediately after the airflow flowed out of the housing from the opening surface.

A decrease in contact angle did not occur with ultraviolet light as in Comparative Example 2. Further, when ozone was generated as in Comparative Example 1, the contact angle decreased. Furthermore, when both ozone generation and ultraviolet irradiation were performed, the contact angle further decreased due to the high reactivity of active oxygen.

In Comparative Example 1, some effects of sterilization, deodorization, and bleaching by ozone were observed, but they were not as good as in Examples 1 to 13. In Comparative Example 2, a certain sterilization effect by ultraviolet rays was observed, but no deodorizing or bleaching effects were observed.

Regarding Example 13, the results of the treatment with active oxygen were good compared to Comparative Examples 1 to 3, but not good compared to Example 1. Specifically, the results of the hydrophilization test and the sterilization test of Example 13 were relatively not good compared to Example 1. The reason for this is thought to be as follows.

That is, in Example 13, the configuration was such that the ozone generator was irradiated with ultraviolet rays. Therefore, it is thought that ozone was decomposed and active oxygen was generated in the vicinity of the ozone generator, which was a position farther away from the object to be treated. Therefore, the concentration of active oxygen reaching the object to be treated decreased, and the degree of treatment with active oxygen decreased relatively, which is considered to be the reason for the above result.

The present disclosure relates to the following configurations and method.

(Configuration 1)
An active oxygen supply device comprising:

   a housing having at least one opening;
   an ozone generator located inside the housing;
   a means for generating an airflow comprising ozone generated inside the housing by the ozone generator, flowing toward the opening and flowing out of the housing through the opening; and
   an ultraviolet light source arranged so that the airflow comprising the ozone and flowing out of the housing through the opening can be irradiated with ultraviolet rays, wherein
   the ultraviolet light source decomposes the ozone in the airflow to generate active oxygen, and
   the airflow comprising the active oxygen generated by irradiating the airflow comprising the ozone with the ultraviolet rays, is supplied to an object to be treated outside the housing.

(Configuration 2)
An active oxygen supply device comprising:

   a housing having at least one opening;
   an ozone generator located inside the housing;
   a means for generating an airflow comprising ozone generated inside the housing by the ozone generator, flowing toward the opening and flowing out of the housing through the opening; and
   an ultraviolet light source arranged so that the airflow comprising the ozone and having flowed out of the housing through the opening can be irradiated with ultraviolet rays, wherein
   the ultraviolet light source decomposes the ozone in the airflow to generate active oxygen, and
   the airflow comprising the active oxygen generated by irradiating the airflow comprising the ozone with the ultraviolet rays, is supplied to an object to be treated outside the housing.

(Configuration 3)
The active oxygen supply device according to configuration 2, wherein the ultraviolet light source is located outside the housing
(Configuration 4)
The active oxygen supply device according to any one of configurations 1 to 3, wherein the ozone generator is a corona discharger equipped with a discharge wire.

(Configuration 5)
The active oxygen supply device according to configuration 4, wherein the means for generating the airflow is a grid electrode arranged closer to the opening than the discharge wire.
(Configuration 6)
The active oxygen supply device according to any one of configurations 1 to 5, wherein the means for generating the airflow is an air blower.
(Configuration 7)
The active oxygen supply device according to any one of configurations 1 to 6, wherein the means for generating the airflow is an air pump introducing air from outside the housing into the housing and creating a positive pressure inside the housing.
(Configuration 8)
The active oxygen supply device according to any one of configurations 1 to 7, wherein the means for generating the airflow is a suction device for gas inside the housing.
(Configuration 9)
The active oxygen supply device according to any one of configurations 1 to 8, wherein a peak wavelength of the ultraviolet rays emitted by the ultraviolet light source is 220 to 310 nm.
(Configuration 10)
The active oxygen supply device according to any one of configurations 1 to 9, wherein the ultraviolet light source is arranged so that the object to be treated outside the housing can be irradiated through the opening.
(Configuration 11)
The active oxygen supply device according to any one of configurations 1 to 10, wherein the ultraviolet light source is an LED or a semiconductor laser.
(Configuration 12)
The active oxygen supply device according to any one of configurations 1 to 11, wherein a flow velocity of the airflow measured at an outlet of the opening when the active oxygen supply device is arranged with the opening thereof facing vertically upward is 0.1 to 100 m/sec.

(Method 13)

A method for treatment by which a surface of the object to be treated is treated with active oxygen, the method comprising:

a step of preparing the active oxygen supply device according to any one of configurations 1 to 12;
a step of placing the active oxygen supply device and the object to be treated at relative positions such that the surface of the object to be treated is exposed when the airflow is caused to flow out from the opening; and
a step of causing the airflow to flow out from the opening to supply active oxygen to the surface of the object to be treated, and treating the surface of the object to be treated with active oxygen.

The present disclosure is not limited to the embodiments described above, and various changes and modifications can be made without departing from the spirit and scope of the present invention. Accordingly, the following claims are appended to set forth the scope of the disclosure.
This application claims priority based on Japanese Patent Application No. 2021-126238 filed on July 30, 2021, and Japanese Patent Application No. 2022-112515 filed on July 13, 2022, the entire contents of which are incorporated herein.

[Reference signs list]

| | |
|---|---|
| 100, 200, 300, 400, 500, 600, 700 | Active oxygen supply device |
| 101 | Housing |
| 103 | Fan |
| 105 | Ultraviolet light source |
| 107 | Airflow |
| 109 | Object to be treated |
| 109-1 | Surface to be treated |
| 111 | Discharge wire |
| 113 | Shield |

| 201 | Grid electrode |
| 301 | Ozonizer (ozone generator) |
| 401 | Mesh member |
| 101-1 | Opening |
| 403 | Air pump |

**Claims**

1. An active oxygen supply device comprising:

   a housing having at least one opening;
   an ozone generator located inside the housing;
   a means for generating an airflow comprising ozone generated inside the housing by the ozone generator, flowing toward the opening and flowing out of the housing through the opening; and
   an ultraviolet light source arranged so that the airflow comprising the ozone and flowing out of the housing through the opening can be irradiated with ultraviolet rays, wherein
   the ultraviolet light source decomposes the ozone in the airflow to generate active oxygen, and
   the airflow comprising the active oxygen generated by irradiating the airflow comprising the ozone with the ultraviolet rays is supplied to an object to be treated outside the housing.

2. An active oxygen supply device comprising:

   a housing having at least one opening;
   an ozone generator located inside the housing;
   a means for generating an airflow comprising ozone generated inside the housing by the ozone generator, flowing toward the opening and flowing out of the housing through the opening; and
   an ultraviolet light source arranged so that the airflow comprising the ozone and having flowed out of the housing through the opening can be irradiated with ultraviolet rays, wherein
   the ultraviolet light source decomposes the ozone in the airflow to generate active oxygen, and
   the airflow comprising the active oxygen generated by irradiating the airflow comprising the ozone with the ultraviolet rays is supplied to an object to be treated outside the housing.

3. The active oxygen supply device according to claim 2, wherein
   the ultraviolet light source is located outside the housing.

4. The active oxygen supply device according to any one of claims 1 to 3, wherein
   the ozone generator is a corona discharger equipped with a discharge wire.

5. The active oxygen supply device according to claim 4, wherein
   the means for generating the airflow is a grid electrode arranged closer to the opening than the discharge wire.

6. The active oxygen supply device according to any one of claims 1 to 5, wherein
   the means for generating the airflow is an air blower.

7. The active oxygen supply device according to any one of claims 1 to 6, wherein
   the means for generating the airflow is an air pump introducing air from outside the housing into the housing and creating a positive pressure inside the housing.

8. The active oxygen supply device according to any one of claims 1 to 7, wherein
   the means for generating the airflow is a suction device for gas inside the housing.

9. The active oxygen supply device according to any one of claims 1 to 8, wherein
   a peak wavelength of the ultraviolet rays emitted by the ultraviolet light source is 220 to 310 nm.

10. The active oxygen supply device according to any one of claims 1 to 9, wherein
    the ultraviolet light source is arranged so that the object to be treated outside the housing can be irradiated through the opening.

**11.** The active oxygen supply device according to any one of claims 1 to 10, wherein the ultraviolet light source is an LED or a semiconductor laser.

**12.** The active oxygen supply device according to any one of claims 1 to 11, wherein a flow velocity of the airflow measured at an outlet of the opening when the active oxygen supply device is arranged with the opening thereof facing vertically upward is 0.1 to 100 m/sec.

**13.** A method for treatment by which a surface of the object to be treated is treated with active oxygen, the method comprising:

a step of preparing the active oxygen supply device according to any one of claims 1 to 12;
a step of placing the active oxygen supply device and the object to be treated at relative positions such that the surface of the object to be treated is exposed when the airflow is caused to flow out from the opening; and
a step of causing the airflow to flow out from the opening to supply active oxygen to the surface of the object to be treated, and treating the surface of the object to be treated with active oxygen.

Fig.1A

Fig.1B

FIG. 2

FIG. 3

FIG. 4

FIG. 5

FIG. 6

EP 4 378 888 A1

FIG. 7

FIG. 8

## INTERNATIONAL SEARCH REPORT

| International application No. |
|---|
| **PCT/JP2022/028763** |

### A. CLASSIFICATION OF SUBJECT MATTER

*C01B 13/02*(2006.01)i; *A61L 2/10*(2006.01)i; *A61L 2/20*(2006.01)i; *A61L 9/015*(2006.01)i; *C01B 13/10*(2006.01)i; *H05H 1/24*(2006.01)i

FI: C01B13/02 Z; A61L2/10; A61L2/20 100; C01B13/10 D; A61L9/015; H05H1/24

According to International Patent Classification (IPC) or to both national classification and IPC

### B. FIELDS SEARCHED

Minimum documentation searched (classification system followed by classification symbols)

C01B13/02; A61L2/10; A61L2/20; A61L9/015; C01B13/10; H05H1/24

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2009-082359 A (IWASAKI ELECTRIC CO., LTD.) 23 April 2009 (2009-04-23)<br>entire text | 1-13 |
| A | JP 2002-316112 A (TOWA CO., LTD.) 29 October 2002 (2002-10-29)<br>entire text | 1-13 |
| A | JP 2006-115973 A (IWASAKI ELECTRIC CO., LTD.) 11 May 2006 (2006-05-11)<br>entire text | 1-13 |
| A | JP 64-025865 A (IWASAKI ELECTRIC CO., LTD.) 27 January 1989 (1989-01-27)<br>entire text | 1-13 |
| P, X | WO 2022/004771 A1 (CANON KABUSHIKI KAISHA) 06 January 2022 (2022-01-06)<br>paragraphs [0015]-[0029], fig. 1, 2 | 1, 2, 9-13 |
| P, A | | 3-8 |

☐ Further documents are listed in the continuation of Box C.　　☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **09 September 2022** | **27 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/028763**

| Patent document cited in search report | | | Publication date (day/month/year) | Patent family member(s) | | | Publication date (day/month/year) |
|---|---|---|---|---|---|---|---|
| JP | 2009-082359 | A | 23 April 2009 | (Family: none) | | | |
| JP | 2002-316112 | A | 29 October 2002 | (Family: none) | | | |
| JP | 2006-115973 | A | 11 May 2006 | (Family: none) | | | |
| JP | 64-025865 | A | 27 January 1989 | (Family: none) | | | |
| WO | 2022/004771 | A1 | 06 January 2022 | JP | 2022-20554 | A | |
| | | | | JP | 2022-13730 | A | |
| | | | | JP | 2022-23131 | A | |
| | | | | WO | 2022/004763 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0125865 B
- JP 2021126238 A
- JP 2022112515 A